# EUROPEAN PATENT APPLICATION

(11) **EP 2 397 172 A1**
(43) Date of publication of application: **21.12.2011**
(21) Application number: 11176949.3
(22) Date of filing: 16.09.2005
(51) Int. Cl.: A61M 5/168, A61M 1/34

(54) **Multichannel coordinated infusion system**

(30) Priority: 17.09.2004 US 943761
(62) Divisional of application: 05797881.9
(71) Applicant: CareFusion 303, Inc., San Diego, CA 92130 (US)
(72) Inventor: Bollish, Stephen J., San Diego, CA California 92129 (US); Vanderveen, Timothy W., Poway, CA California 92064 (US)
(74) Representative: Richards, John

(57) **Abstract**

An infusion system comprising:
a first delivery unit that delivers an infusion ultrafiltrate to a hemofilter;
a first measurement device that determines an amount of infusion ultrafiltrate in the infusion system;
a receiver unit that receives a patient's removed ultrafiltrate from the hemofilter; a second measurement device that determines an amount of removed ultrafiltrate in the infusion system; and
a control system communicating with the delivery and receiver units and the first and second measurement units to determine optimum flow rates to and from the hemofilter, based upon the determined amounts of infusion ultrafiltrate and removed ultrafiltrate in the infusion system, and a predetermined desired pressure differential between the infusion and removed ultrafiltrates, and for controlling the delivery and 15 receiver units accordingly.

## Description

### FIELD OF THE INVENTION

This invention is generally related to monitoring the amount of medical fluid delivered by a pump, and more particularly, to accurately monitoring the amount of medical fluid remaining and calculating a flow rate for complete delivery in a predetermined time period.

### BACKGROUND OF THE INVENTION

It is known for infusion systems that contain multiple infusion pumping modules to include a centrally managed infusion pump system in which pump and monitoring modules are selectively attached to a central management unit. The central management unit controls the internal setup and programming of the attached modules, and receives and displays information from them. Each module is capable of being detached from the central management unit.

Due to inaccuracies of the infusion system, that can be plus or minus 5% or more over long periods, and other factors such as intravenous (IV) bag overfill or interruptions in flow, a desired volume of a drug may not be infused within a desired period and the infusion may be ahead or behind schedule by, in some cases, an hour or two. The clinician, i.e. a person qualified in the clinical practice of medicine, psychiatry, or psychology, must then manually increase or decrease the flow rate in order to compensate for these factors as soon as the problem is recognized. Interruptions in receiving medication can result in inconvenience and delay for the patient and clinicians, as well as potentially negative therapeutic efficacy of the medication. Drug toxicity may also become a problem where the infusion rate is increased toward the end of the infusion to assure on-time completion.

One approach clinicians use to deal with the above problem is to weigh the IV bag to determine the exact volume of the bag. This is not a fully satisfactory approach, since the bag must be carefully weighed in the pharmacy and the empty weight of the bag subtracted from the total weight of the bag. Differences in accuracy of the scale and correction for the specific gravity (density) of the solution (which is usually not known) result in additional inaccuracies. Typically, a clinician sets an infusion flow rate based upon the derived volume of the solution. This method does not allow for accurate correction of flow rate due to interruptions in flow once the infusion is initiated and thus may not deliver the infusion medication to the patient over the desired time period. At the completion of an infusion that has been subjected to interruptions, the clinician is often left with a sizable volume of residual medication remaining in the IV bag or tubing that must be flushed or discarded. If the residual volume is discarded, the patient may not receive the full dose intended by the clinician, thus reducing the medication's effect.

Therefore, there has been identified a continuing need to provide a medical infusion system that will accurately infuse a medication over predetermined periods of time.

### SUMMARY OF THE INVENTION

Briefly and in general terms, the present invention is directed to an infusion system that delivers a first infusate, preferably a medication, to an IV line, and a second infusate, preferably a neutral carrying solution, to the patient's IV line. The infusion system also includes a measurement device that determines an amount of undelivered first infusate remaining in the system. Furthermore, the infusion system includes a control system that controls delivery of the first infusate, and that enables input of a predetermined volume of infusate to be delivered, and input of a time period within which infusion of the the first infusate to be delivered, and input of a time period within which infusion of the first infusate volume should occur. The control system also communicates with first and second delivery devices and the measurement device to determine an optimum first infusate infusion flow rate based on the predetermined first infusate volume, the predetermined infusion time period, and the determined amount of undelivered first infusate remaining in the system, and controls the first delivery device to deliver the first infusate at an optimum infusion flow rate.

In accordance with another aspect of the invention, there is further provided an infusion system comprising a first delivery device that delivers an infusion ultra filtrate to a hemofilter, and a first measurement device that determines the amount of infusion ultrafiltrate in the infusion system. The infusion system also includes a second delivery device that receives a patient's removed ultrafiltrate from the hemofilter, and a second measurement device that determines an amount of removed ultrafiltrate in the infusion system. Furthermore, the infusion system includes a control system communicating with the first and second delivery devices and the first and second measurement units to determine optimum flow rates to and from the hemofilter, based upon the determined amounts of infusion ultrafiltrate and removed ultrafiltrate in the infusion system, and a predetermined desired pressure differential between the infusion and removed ultrafiltrates, and for controlling the first and second delivery units accordingly.

In other aspects the invention, there is provided a method of infusing a first infusate into a patient. The clinician or the system itself determines the total volume of first infusate that is to be infused into the patient. The clinician then ascertains the period of time over which it is desired that the total volume of first infusate is to be infused, such as twenty-four hours. A position sensor of a syringe pump or the weight cell of a large volume pump detects the remaining amount of first infusate to be infused. A control system constantly calculates the time remaining in the predetermined period. The large volume pump (LVP) delivers the second infusate into the patient's infusion line while. either a syringe pump or a LVP and weight cell combination infuses the first infusate into the IV line. The control system preferably constantly and automatically adjusts the infusion flow rate based on the remaining amount of first infusate to be infused and the time remaining in the predetermined time period. The detecting, calculating, delivering, infusing, and adjusting steps are preferably repeated until the total volume of first infusate is infused.

In yet other aspects, there is provided a method of infusing an ultrafiltrate into a patient comprising the steps of delivering an infusion ultrafiltrate to a hemofilter, measuring the amount of infusion ultrafiltrate in the infusion system, receiving a patient's removed ultrafiltrate from the hemofilter, measuring the amount of removed ultrafiltrate in the infusion system, and adjusting flow rates of the delivering and the receiving steps based upon the measured amounts of infusion and removed ultrafiltrate in the infusion system and a predetermined desired pressure differential between the infusion and removed ultrafiltrates.

The novel features of this invention, as well as the invention itself, both as to its structure and its operation, will be best understood from the accompanying drawings, taken in conjunction with the accompanying description.

### BRIEF DESCRIPTION OF THE DRAWINGS

FIG. 1 is a schematic view of a multichannel coordinated infusion system in accordance with aspects of the present invention showing medical fluids from two different sources of fluid, a receptacle (fluid bag) and a syringe, being pumped into a common IV administration line for infusion into a patient by two infusion pumps under the common control of a programming unit;
FIG. 2 is a enlarged view of the programming unit shown in FIG. 1;
FIG. 3 is graph showing flow rate versus time in an infusion session;
FIGS. 4A to 4V are user interface screens presented by the programming unit of FIG. 1 when programming the infusion from the two different sources shown in FIG. 1;
FIG. 5 is a flow chart of a method of infusing a medication into a patient in accordance with the infusion system of FIGS. 1-4;
FIG. 6 is a schematic view of another embodiment of a multichannel coordinated infusion system in accordance with aspects of the present invention showing medical fluids located in two different sources of fluid and a large volume infusion pump having a weight cell; the fluids being pumped into a common IV line for infusion into a patient under the common control of a programming unit;
FIG. 7A to 7U are user interface screens presented by the programming unit of FIG. 6, when programming the infusion from the two different sources shown in FIG. 6;
FIG. 8 is a schematic view of a further embodiment of a multichannel coordinated infusion system in accordance with aspects of the present invention, showing removal and replacement of ultrafiltrates through large volume infusion pump-weight cell combinations, for infusion in a patient under the common control of a programming unit; and
FIG. 9 is a flow chart of a method of removing and replacing ultrafiltrates in accordance with the infusion system of FIG. 8.

### DESCRIPTION OF THE PREFERRED EMBODIMENTS

Referring now to the drawings with more particularity, in which like reference numerals refer to like or corresponding elements among the several views, the multichannel coordinated infusion system according to the invention infuses a predetermined volume of medication over a specific predetermined time period, such as 1000 ml of a chemotherapeutic agent over a 24 hour period.

The present invention will be explained by way of example whereby a patient undergoes a chemotherapy medication or other medication infusion. It should be understood that such an example is for illustrative purposes only.

Typically, a chemotherapy medication is infused over multiple sessions lasting many days. Due to inaccuracies in some infusion systems or interruptions in the delivery of an infusion, the infusion may be early or late by many hours by the end of the infusion session. The present invention addresses this problem and illustrative embodiments are provided below. In one embodiment, an accurate fluid transferring device, for example a weight cell in combination with a large volume infusion pump ("LVP") is used where the fluid transferring device is controlled by an intelligent control module.

Syringe pumps typically consist of a cylinder that holds a fluid that is expelled by an advancing plunger. The plunger is usually advanced into the cylinder by a drive mechanism that includes a motor connected through a gear or gears to a driver head to provide a relatively constant pulse-free flow. In accordance with an aspect of the present invention, a means is provided for accurately infusing a predetermined volume of fluid over a predetermined period. Rather than programming a specific flow rate, a clinician may program a specific volume to be infused over a specific period and the system automatically adjusts the flow rate, within certain parameters, based on the remaining volume to be infused and the remaining period for infusion. The system also accommodates for any changes in starting and stopping of the system due to circumstances that might occur during infusion such as an occlusion or air in the infusion line or for some other reason where the infusion needs to be momentarily stopped.

Typically, medication solutions are provided in concentrated form, and must be diluted prior to infusion into the patient. As syringes generally contain relatively small volumes of fluid, as compared to IV drip bags, many syringe-borne medications are provided in concentrated form and must be diluted during administration. The present invention allows concentrated medications in a syringe to be diluted on a continuous basis by a neutral carrying solution such as normal saline solution supplied via a large volume pump (LVP).

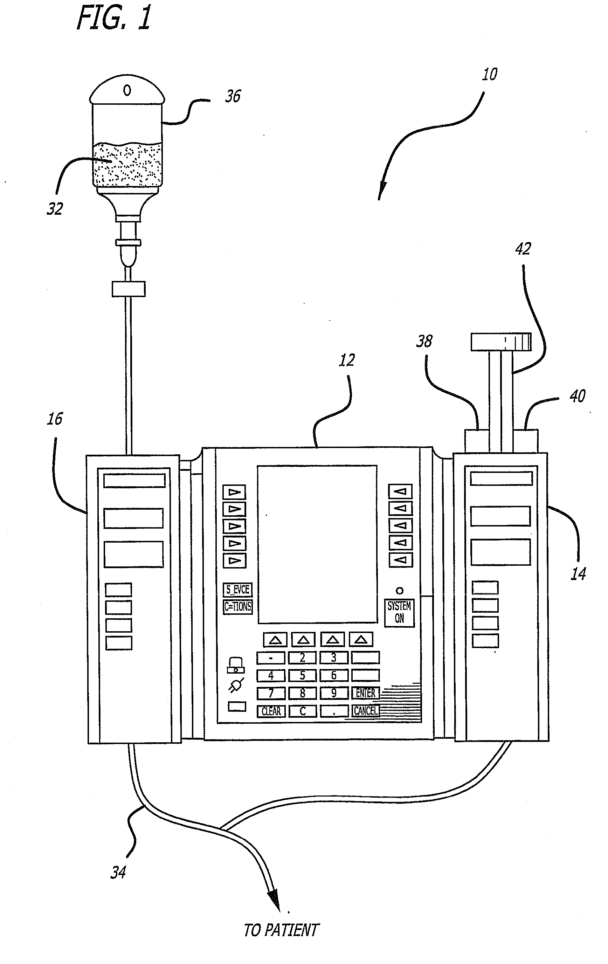

FIG. 1 is a view of a multichannel coordinated infusion system 10 according to one embodiment of the invention. The multichannel coordinated infusion system 10 incorporates a programmable patient care system similar to that disclosed in U.S. Patent No. 5,713,856 to Eggers, issued February 3, 1998, which is incorporated herein by reference. The programmable patient care system includes a patient care unit (PCU) 12 in combination with at least two functional units 14 and 16. The PCU 12 incorporates a control system and generally performs four functions in the patient care system: it provides a physical attachment of the system to structures such as IV mounting poles and bed rails; it provides power to the system; it provides an interface between the system and external devices, and, except for certain information; and it provides a majority of the user interface with the system.

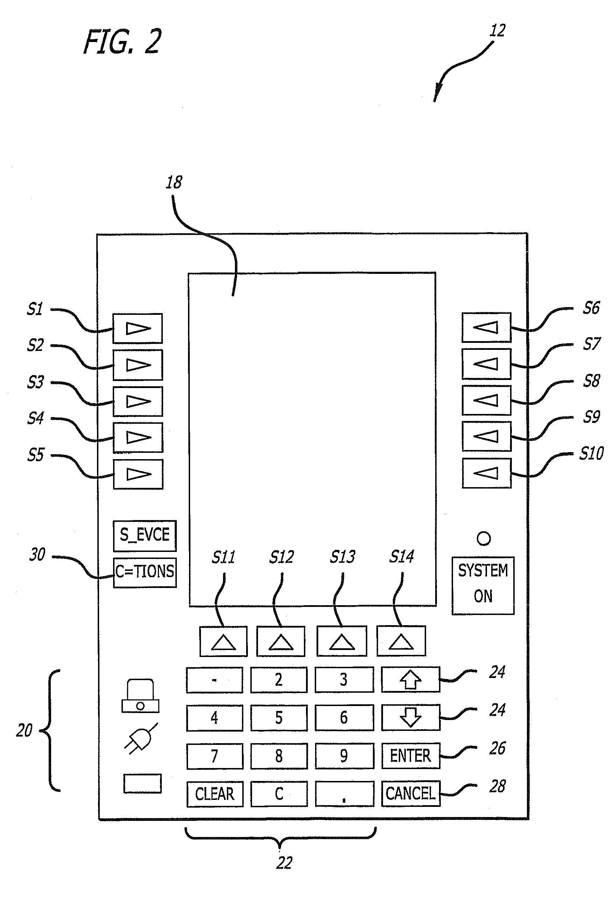

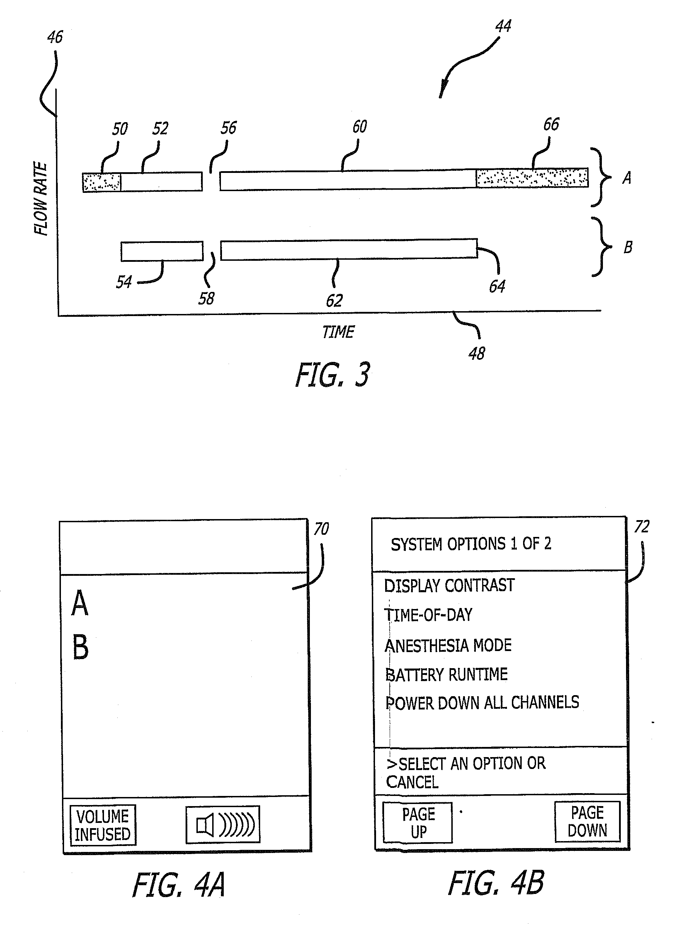

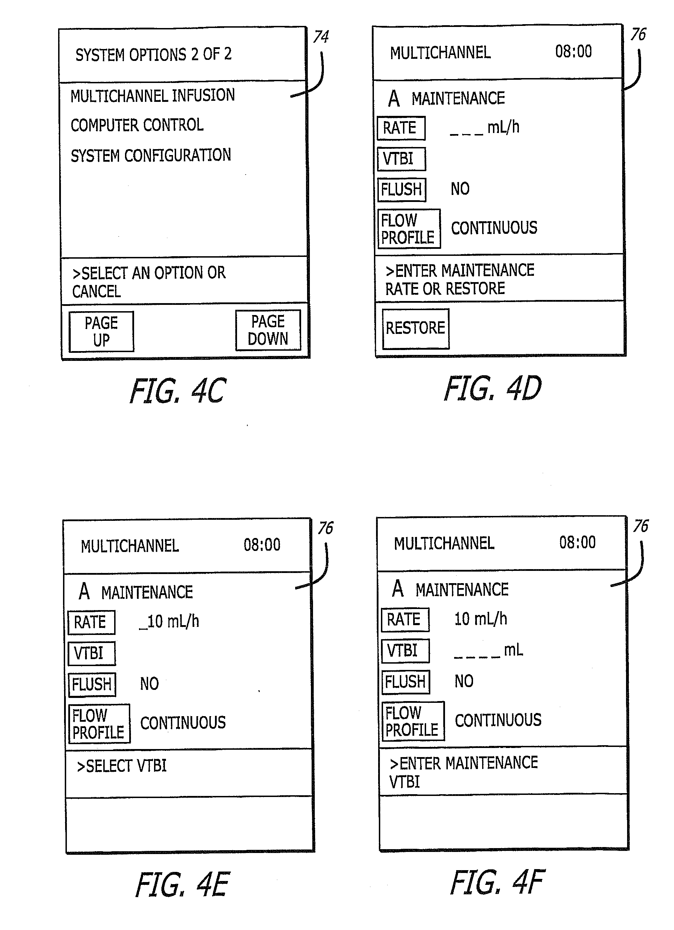

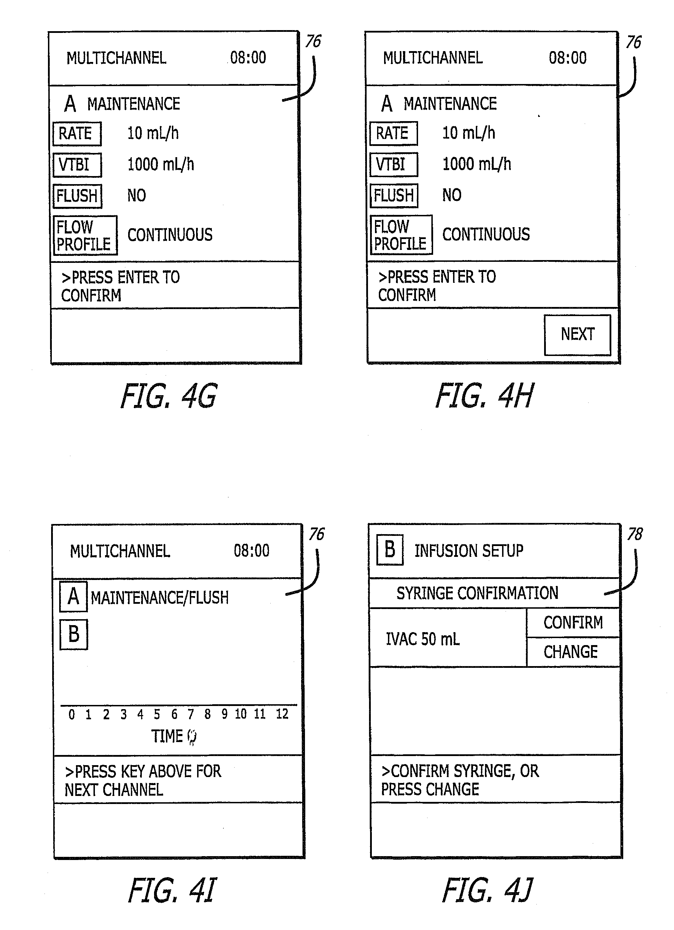

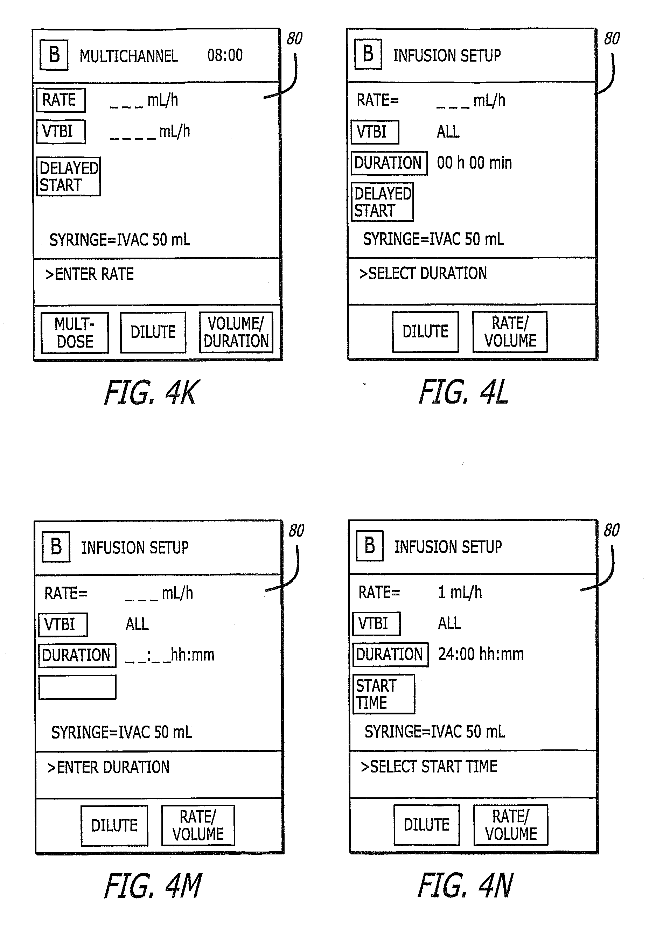

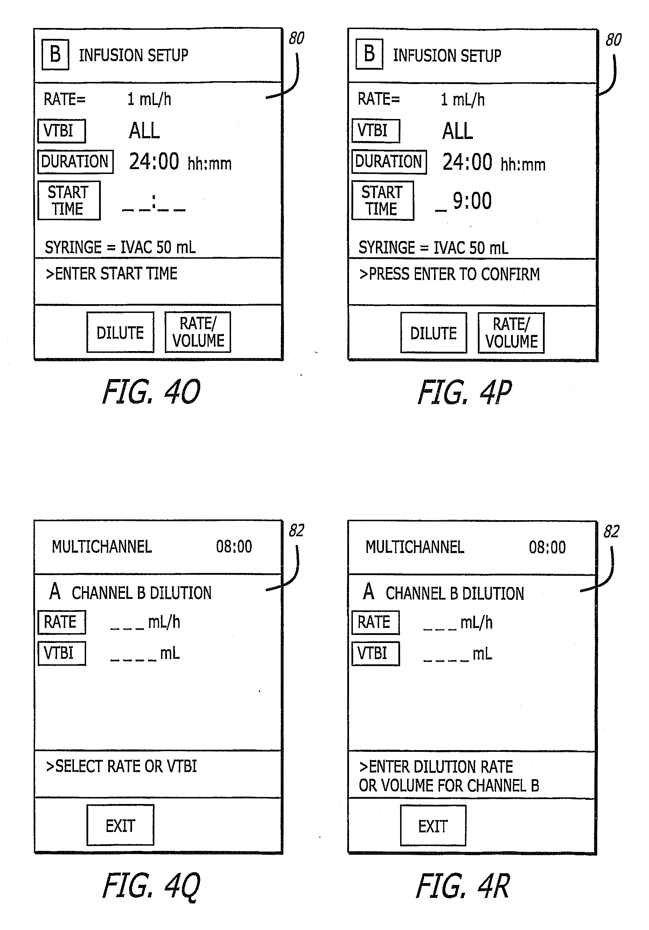

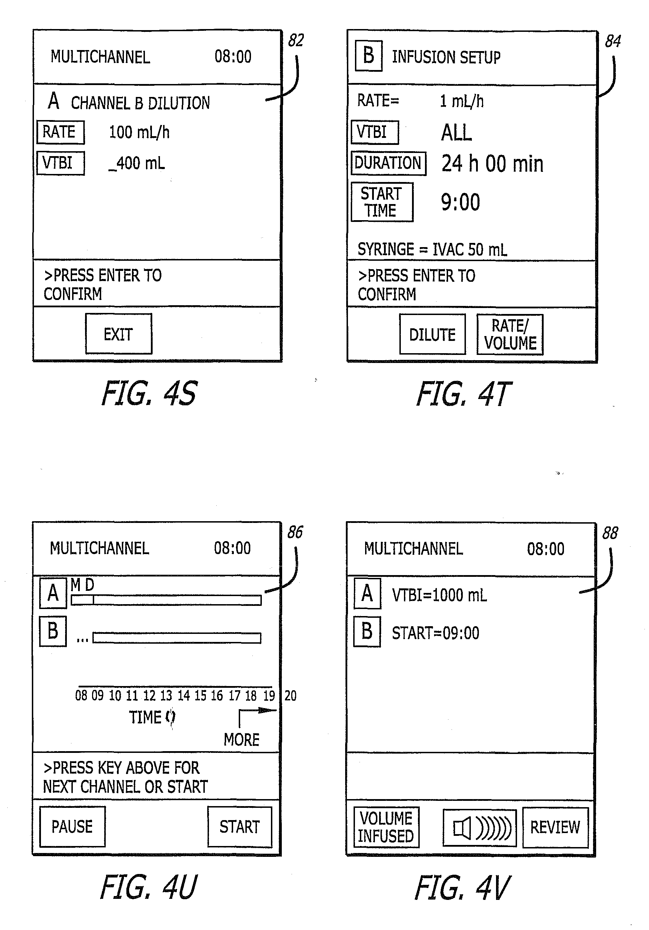

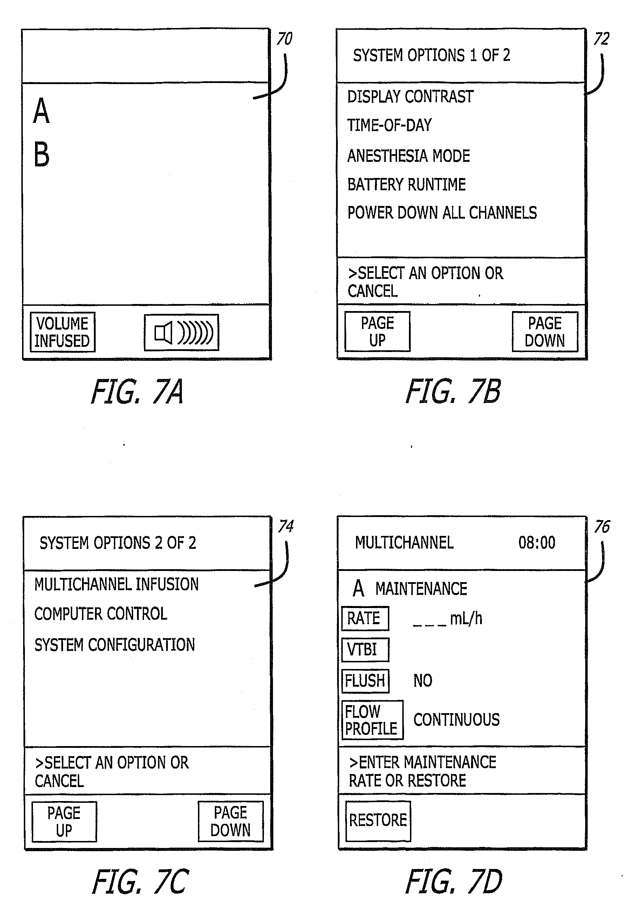

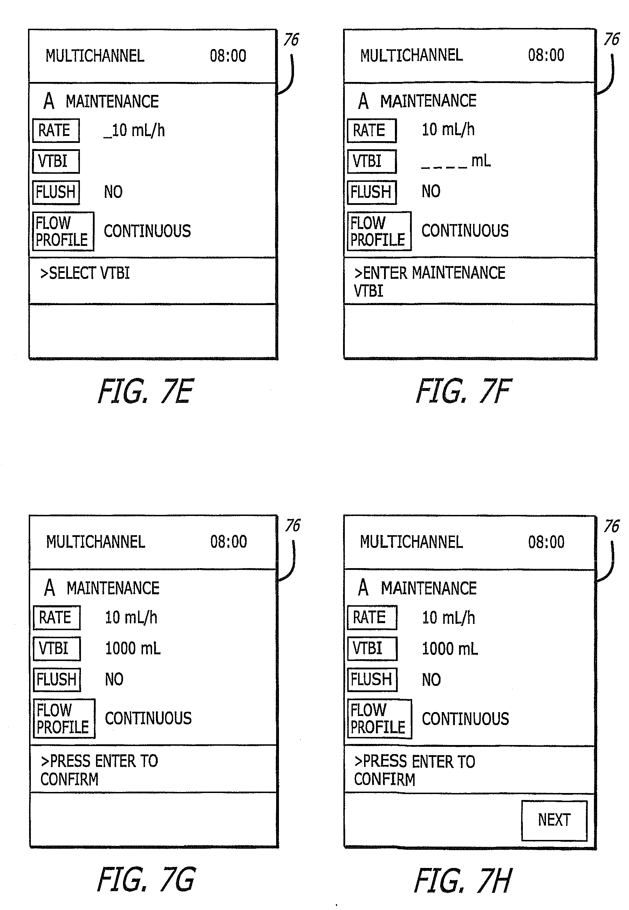

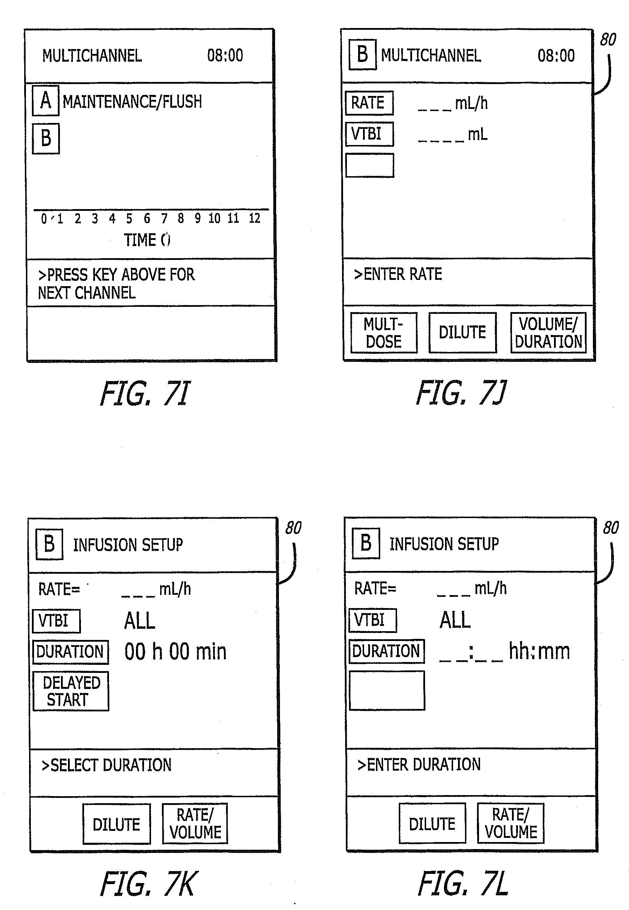

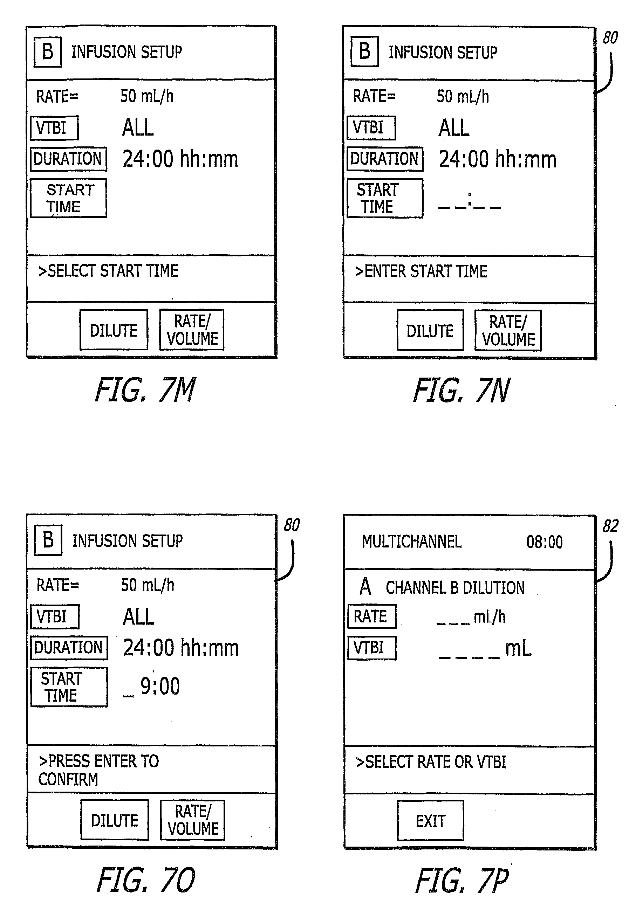

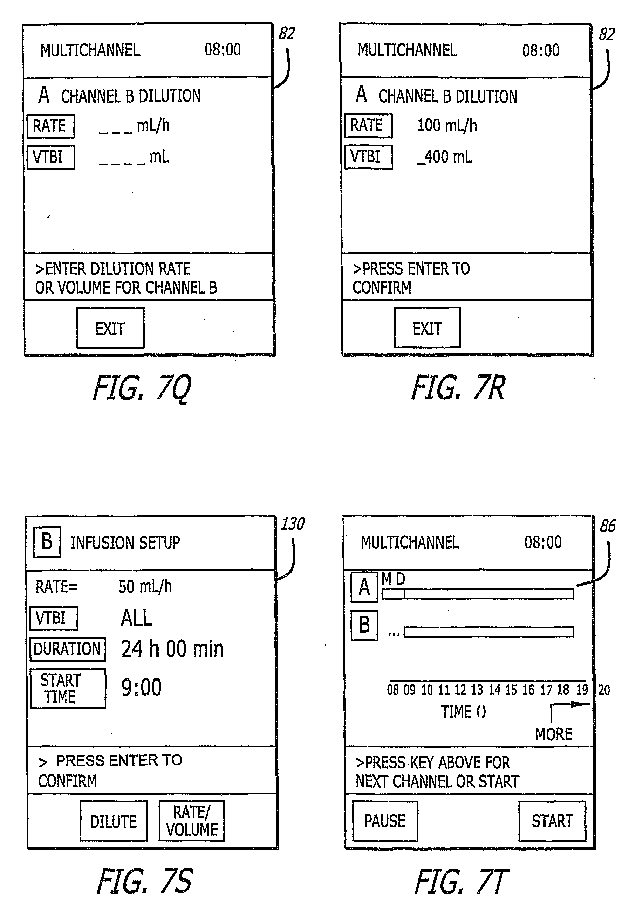

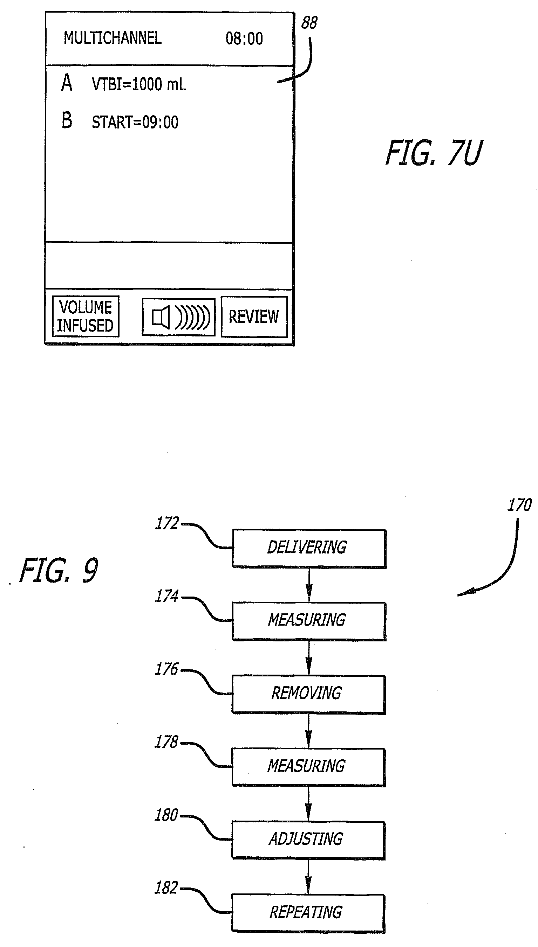

FIG. 2 is an enlarged view of the PCU 12 shown in FIG. 1. The PCU 12 includes an information display 18 that may be any type of display such as a liquid crystal display. The display 18 may be used during setup and operation procedures to facilitate data entry and editing. The display 18 may also be used to display various operating parameters as described in relation to FIGS. 4 and 7. The PCU 12 may furthermore contain a plurality of hard keys 20 and softkeys S1-S14 for entering data and commands. Hard keys 20 may include numerical hard keys 22, navigation keys 24 (such as up and down keys), an ENTER key 26, a CANCEL key 28, and an OPTIONS key 30. Other common keys exist such as POWER and SILENCE used to terminate an audible alarm. The numerical hard keys 22 may partly be used for entering numerical data, while the remainder of the hard keys, as well as the softkeys S1-S14, may be used for entering operational commands. In this embodiment there are provided five softkeys S1-S5 on the left of the display 18, five soft keys S6-S10 on the right of the display 18, and four softkeys S11-S14 under the display 18.

Again referring to FIG. 1, there are two functional units 14 and 16 mounted to the PCU 12. The functional units 14 and 16 are preferably removably attached to the PCU 12 and may be interchanged with other functional units as described in U.S. Patent No. 5,713,856. It is to be understood that although two functional units 14 and 16 are shown in FIG. 1, a different number of functional units may be incorporated into the patient care system 10.

The first functional unit 16 is preferably an infusion pump or the like. More specifically, the first functional unit 16 is preferably a large volume parenteral pump ("LVP") with a flow rate of approximately 1000 to 2000 ml/hr, or more. The primary task of the first functional unit 16 in this embodiment is to deliver a dilution solution 32 into an IV line 34 inserted into a patient via a dilution infusion channel (Channel A), in this instance, the LVP 16. The dilution solution may be any neutral solution such as a normal saline solution, and is typically contained within a receptacle such as an IV bag 36.

The second functional unit 14 is preferably a low-volume timed infusion pump, such as a syringe pump. The primary task of the second functional unit 14 in this embodiment is to deliver a concentrated medication into the IV line 34 via a medication infusion channel (Channel B), in this instance, a syringe pump 14. The medication may be any type of drug in fluid form, for example a chemotherapeutic agent.

The syringe pump 14, according to one embodiment, includes a syringe size detection system having a sensor 38, such as a linear actuator, to measure the syringe diameter that is then used to determine, using a look-up table or the like, the type of syringe that is being used. For example a syringe having twenty mm diameter may have a fifty ml volume. There may however be other syringes with the same diameter, so, according to one embodiment, a clinician is prompted to confirm the syringe size as will be discussed in relation to FIG. 4J.

The syringe pump 14 may furthermore include a measurement device 40 such as an accurate linear position sensor that ascertains how far a plunger 42 of a syringe has traveled and how much farther the plunger 42 must travel to reach the end of its delivery stroke. The plunger position taken together with the syringe size, enable a processor within the multichannel coordinated infusion system 10 (see US Patent No. 5,713,856) to accurately determine the volume of medication remaining in the syringe. Such a determination can be made continuously in real time if desired.

In use, the control system of the PCU 12 communicates with the functional units 14 and 16, and the measurement device 40 to determine an optimum first infusate infusion flow rate based on a determined amount of undelivered first infusate remaining in the system, a predetermined volume of fluid to be infused, and a predetermined infusion time period. The control system of the PCU 12 then controls the flow rates of the functional units 14 and 16 accordingly.

FIG. 3 is a graph 44 of flow rate 46 versus time 48 in a typical infusion session. Channel A is typically set up to infuse a pharmacologically inert (neutral) or carrier solution such as a saline solution at a maintenance flow rate until the medication is loaded and infusion begun. Typically, Channel A, the dilution channel, is continuously run to keep the IV line "open", although this is not a prerequisite. This initial flow of dilution is referred to as a maintenance infusion 50. A different predetermined dilution flow rate 52 on Channel A is set to begin infusing simultaneously with the start of a medication infusion 54 on Channel B. If the infusion of either Channel A or Channel B is interrupted 56 or 58 for any reason, both Channels A and B will stop simultaneously. When the channel that initially stopped is restarted, both channels restart pumping simultaneously 60 and 62. The PCU 12 automatically adjusts the rate of the medication infusion (Channel B) so as to infuse the remainder of a desired predetermined medication volume within the remainder of an allotted predetermined period. Once the desired medication has completely been infused, the medication infusion channel automatically stops 64. The dilution channel (Channel A) may however continue to infuse a maintenance infusion 66.

FIGS. 4A to 4V are interface screens used in connection with an embodiment of the invention. Using FIGS. 4A to 4V, an example of a user interface and control system will now be described.

FIG. 4A is a main programming interface screen 70. This main programming screen data 70 is displayed when the infusion system is either running or when the system is dormant or not running. As a general rule, pressing a soft key (S1-S14 of FIG. 2) next to a displayed function will activate the function or command displayed next to it on the display screen. "A" and "B" are indicative of the Channel A and Channel B infusion modules connected to the system. If more modules are connected to the system, an additional letter would be displayed for each module. The "volume infused" or "alarm loudness" functions or commands may also be accessed via the soft keys. By pressing the "Options" hard key (reference numeral 30 in FIG. 2) the screen of options 72 of FIG. 4B is displayed. FIG. 4B is a first options interface screen 72. A number of options concerning general operation are displayed on the screen. By pressing the "PAGE DOWN" soft key S 14 (FIG. 2), a second options screen 74 is shown as per FIG. 4C.

Options 74 more particularly relevant to infusion are presented in FIG. 4C. By pressing the soft key S1 next to "Multichannel Infusion" the functions pertaining to further aspects of the present invention may be accessed.

FIG. 4D presents an interface 76 for Multichannel Infusions. This interface relates to the setup of the maintenance flow of the saline solution via Channel A. The system defaults to entering the flow rate for the maintenance infusion. The clinician may then enter the desired flow rate in milliliters per hour (ml/h) as shown in FIG. 4E. For example, a maintenance flow rate of 10 ml/h is entered via the numerical hard keys. By pressing the soft key next to "VTBI" ("volume-to-be-infused") the interface screen 76 of FIG. 4F will be displayed. The clinician may enter an exact or approximate volume of maintenance fluid to be infused, such as 1000 ml as shown in FIG. 4G. The soft keys next to "Flush" or "Profile" may be pressed to alter either of these settings. The "ENTER" hard key (reference number 26 in FIG. 2) may be pressed to confirm the variables that were input. FIG. 4H is the interface screen 76 of a confirmation. The clinician is again prompted to confirm the variables as a backup precaution. The "ENTER" hardkey is again pressed to confirm.

FIG. 4I is a summary screen. Here, Channel A is shown to be delivering the maintenance infusion. To move now to the medication Channel B, the soft key S2 located adjacent the "B" Channel screen indication is pressed, displaying the interface shown in FIG. 4J. FIG. 4J is a syringe confirmation screen. A feature specific to the syringe pump embodiment of the invention as shown in FIG. 4J is that the clinician must identify the type of syringe being used (as flow rate is dependent upon the rate of travel of the plunger and the diameter of the syringe barrel). The syringe type may be automatically determined by the pump, as discussed above, and are displayed as per this example; i.e., an IVAC 50 ml syringe. The soft keys S7 or S8 may then be pressed to either confirm or change the detected syringe type. If the syringe type is incorrect, an interface screen from which the user may select a different syringe type will be displayed (not shown). If the syringe type is confirmed, the interface shown in FIG. 4K is displayed.

The clinician is prompted to enter the medication infusion flow rate, which may be entered via the numerical hard keys. If the clinician would prefer to infuse a specific volume or the entire volume of the syringe, the soft key S2 next to "VTBI" may be pressed. The clinician may either enter a volume to be infused, or, by either pressing the hard up or down keys (numeral 208 of FIG. 2) or the soft key S2 again, the clinician may select to infuse the entire contents of the syringe. Infusing the entire contents of the syringe is evidenced by "ALL" next to "VTBI" as shown in FIG. 4L. The "ALL" setting may alternatively be set as a default setting. Pressing the S3 soft key will select the option to input the duration of this infusion as shown in FIG. 4M.

According to this embodiment of the invention, the clinician typically requires infusing a predetermined volume of medication over a predetermined time period. As described in relation to FIG. 4L, the clinician has selected to infuse the entire volume of the syringe. To input the period over which the entire volume is to be infused, the clinician enters the desired time via the numerical hard keys, for example a 24 hour period as shown in FIG. 4N. By pressing the S4 soft key the start time of the infusion may be entered as shown in FIG. 40.

In FIG. 4P, the clinician has entered the desired start time of the infusion as 9:00. The system may alternatively be set to default to the current time and the clinician can utilize the up or down hard keys to change the start time. A delay in infusing the medication may be desirable in order to allow the clinician time to first perform another task, such as administering a pre-medication such as an anti-nauseant drug, prior to giving the chemotherapeutic medication via another syringe or infusion channel.

As described above, the medication typically must be diluted with a neutral solution, such as saline, prior to infusion into the patient. To set up the dilution channel the clinician presses the S12 soft key associated with "DILUTE", which displays the dilution interface screen shown in FIG. 4Q. To enter the flow rate of the dilution solution the clinician presses the S2 soft key associated with "RATE", shown in FIG. 4R. A flow rate, for example 100 ml/h, may be entered via the numerical hard keys. By pressing the S3 soft key associated with "VTBI", the clinician may enter the approximate volume ot dilution solution to be infused, for example 400 ml, as shown in FIG. 4S. Only an estimate is required as the more important parameter is the flow rate of the dilution solution. If during the infusion the system runs out of dilution solution, an alarm will sound and both Channel A and B will simultaneously stop until the dilution solution is replaced and the system restarted. To complete the dilution solution setup, the clinician presses the "ENTER" hard key (reference numeral 26 of FIG. 2). The dilution solution is infused simultaneously with the medication, and therefore the start time and duration are the same for both the dilution Channel A and the medication infusion Channel B.

FIG. 4T is an interface screen 84 for the Channel B setup where the clinician is prompted to press the "Enter" hard key to confirm the setup. By pressing "Enter", the interface 86 shown in FIG. 4U is displayed. FIG. 4U shows the infusion of both Channel A and B on a time line. Soft keys associated with either channel may be pressed to view or change that channel's setup. If additional channels were connected to the system they too would be displayed. To start the system the clinician presses the S14 soft key below "START". Once the system has started running, the summary screen 88 in FIG. 4V is displayed. As per the example, the maintenance flow will immediately start infusing and the medication together with the dilution solution will only begin to infuse at 9:00. After 9:00 the screen will change to show that the entire contents of the syringe are being infused and the duration for infusion would decrement over time.

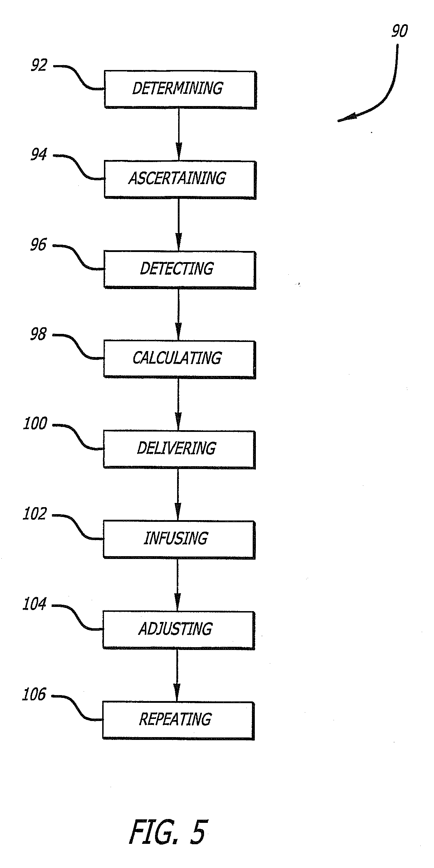

FIG. 5 is a flow chart of a method 98 of infusing a medication into a patient in accordance with the infusion system described above and shown in FIGS. 1-4. The clinician or the system itself, as described above, determines at step 90 the total volume of medication that is to be infused into the patient that may simply be to infuse the entire contents of an IV drip bag or syringe. The clinician then selects at step 94 the period in which the total volume of medication is to be infused, such as twenty-four hours. The linear position sensor 40 constantly detects at step 96 the remaining amount of medication to be infused. The control system constantly calculates at step 98 the time remaining in the predetermined period. The pump delivers at step 100 the neutral carrier solution into an IV line while the syringe pump infuses at step 102 the medication into the infusion circuit. The control system constantly and automatically adjusts at step 104 the Channel B infusion flow rate based on the remaining amount of medication to be infused and the time remaining in the predetermined period. The detecting, calculating, delivering, infusing, and adjusting steps 92-104 are repeated at step 106 until the total volume of medication is infused.

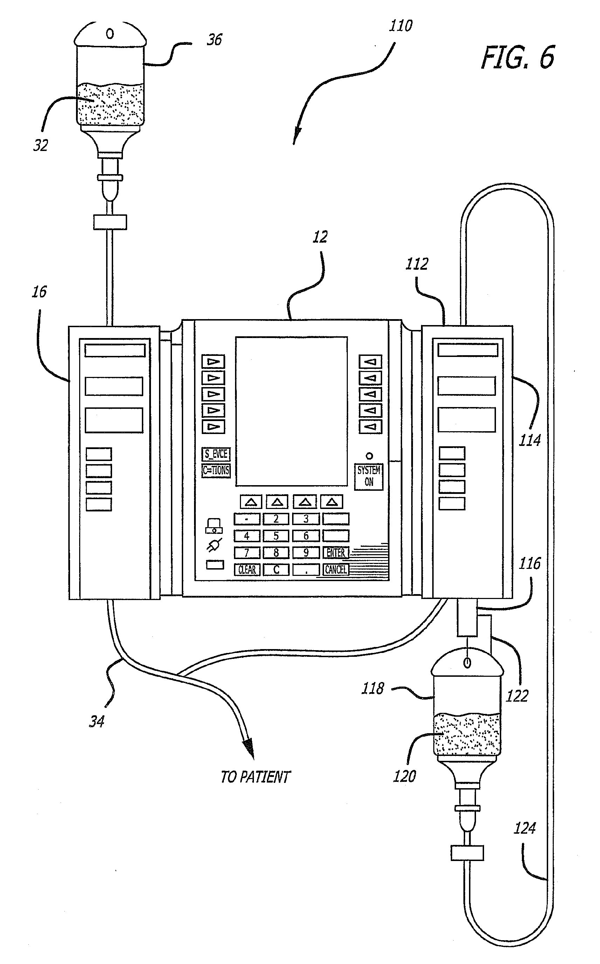

FIG. 6 presents a multichannel coordinated infusion system 110, according to another embodiment of the invention. The multichannel coordinated infusion system is similar to the syringe pump embodiment described above. As in the prior embodiment the present multichannel coordinated infusion system comprises a PCU 12, including a control system, and a LVP 16. Dilution solution 32 contained within an IV bag 36 is pumped through Channel A by the LVP into an IV line 34 connected to a patient. The system further includes a functional unit 112 that comprises a LVP 114 and a weight cell 116. The weight cell communicates via wired or remote communication (e.g. infrared, Radio Frequency) with the PCU 12.

In use, a receptacle 118, such as an IV drip bag, of unknown volume but having a known empty or tare weight containing a medication 120 is suspended from weight cell 116. A clinician typically identifies the receptacle to be suspended from the weight cell (e.g. a commercially available 250 ml plastic solution bag), and enters this information together with the approximate volume and duration of the infusion into the PCU 12. The type of receptacle and hence its tare weight may be determined automatically by a receptacle detection system 122. The tare weight may be determined from a database in the PCU programmed with the names of manufacturers of receptacles and the tare weight of their receptacles, or the manufacturer may include a bar code or another information device on its receptacle that the PCU may be able to read. The pharmacist may weigh the dry bag and put in a bar code or other electronic tag, or the manufacturer may include an identifier on the receptacle including the manufacturer's name, model number, and weight of the bag in grams. The weight of the fluid in the receptacle is calculated by subtracting that specific container's known tare weight from the receptacle's net weight measured by the weight cell. A tube 100 delivers the medication of the bag through the LVP 114 (Channel B) to connect with the IV line 34.

The processor within the multichannel coordinated infusion system 110 (control system) automatically determines a precise infusion flow rate based on the predetermined total volume to be infused, the measured fluid weight, and the time remaining in the period allotted for the infusion. This determination may be made continuously and in real time if desired. Inaccuracies due to different specific gravities of fluid are generally minimal (as most medication fluids have specific gravities close to one, i.e. a similar mass to water which is used as a standard) but may be taken into account by the multichannel coordinated infusion system 110. The system therefore automatically determines the weight change of the receptacle 118 and adjusts the medication infusion rate via Channel B accordingly, to insure complete delivery of the contents of the receptacle, or a specified volume, in the predetermined time period allotted for the infusion. In doing so, the system automatically adjusts for periods of no flow due to alarms, pause conditions, etc.

The functional units 16 and 114 are preferably removably attached to the interface unit PCU 12 and may be interchanged with other functional units, as described above. It is to be understood that although two functional units are shown in FIG. 6, a different number of functional units may be incorporated into the patient care system 110.

Typically LVPs need a positive head height to operate accurately. In the weight cell embodiment discussed above, the constant control of the flow rate through Channel B negates the need for a positive head height. The arrangement above permits the delivery of the entire contents of an unknown volume in an exact predetermined period of time. It also results in the ability to deliver jointly controlled dilution and infusion solutions.

FIGS. 2 and 3 are equally applicable to the weight cell embodiment described above.

FIG. 7A to 7U are user interfaces used in connection with the above weight cell embodiment of the invention. The PCU used in connection with this embodiment is programmed in a similar manner to that explained in relation to the prior embodiment. Access to the multichannel infusion mode of the present invention is attained as described in relation to FIGS. 4A to 4C and shown in FIGS. 7A to 7C for the sake of completeness. A Maintenance flow rate for Channel A is set as described in relation to FIGS. 4D to 4H and shown in FIGS. 7D to 7H for the sake of completeness. A summary screen where Channel A is shown to be delivering a maintenance infusion is shown in FIG 7I. By pressing the S2 soft key next to "B" the interface screen 80 for the infusion setup is displayed as per FIG. 7J.

In a similar manner to that described in relation to FIGS. 4K to 4P, and shown in FIGS. 7J to 70 for the sake of completeness, the flow rate "RATE", volume to be infused "VTBI", duration of the infusion "DURATION" and the start time of the infusion "START TIME" are entered into the PCU 80. The programming of the flow rate is not critical to the system if the entire volume of the receptacle is to be infused as evidenced by "ALL". This is because the flow rate is constantly calculated by the multichannel coordinated infusion system that divides a calculated fluid weight by the number of hours and minutes remaining in an allotted period for the infusion. The weight of the infusion fluid in the receptacle is calculated by subtracting that specific container's known tare weight from the receptacle's net weight measured by the weight cell.

The Channel A dilution infusion is then set as described in relation to FIGS. 4Q to 4S and shown in FIGS. 7P to 7R for the sake of completeness. An infusion setup confirmation screen 130 is then presented to the clinician as per FIG. 7S. The clinician is prompted to press "ENTER" to confirm the setup shown in FIG. 7S. On pressing "ENTER", the interface screen 86 shown in FIG. 7T is displayed. FIG. 7T shows the infusion of both Channel A and B on a time line. Soft keys associated with any channel may be pressed to view or change that channel's setup. If additional channels were connected to the system they too would be displayed. To start the system the clinician presses the S14 soft key below "START". Once the system has started running, the summary screen 88 in FIG. 7U is displayed. As per the example described in relation to the prior embodiment, the maintenance flow will immediately start infusing and the medication together with the dilution solution will only begin to infuse at 9:00. After 9:00 this screen will change to show that the entire contents of the receptacle are being infused and the duration would decrement over time.

The method of infusing a medication into a patient in accordance with the infusion system of FIGS. 6-7 is the same as that explained in relation to the prior embodiment, except that the position sensor of the weight cell constantly detects at step 96 the remaining amount of medication to be infused, and the LVP and weight cell combination infuses at step 102 the medication into the infusion circuit.

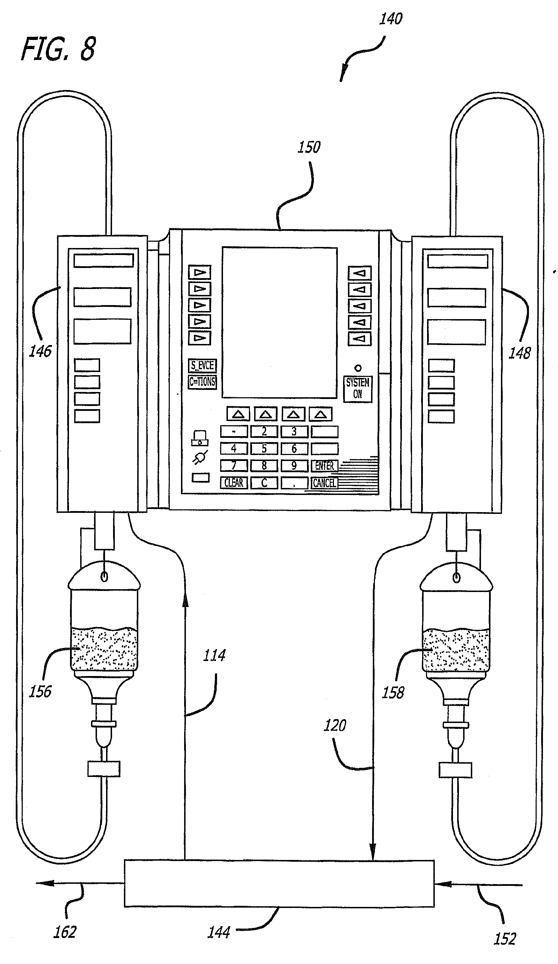

FIG. 8 presents a multichannel coordinated infusion system 140 according to a further embodiment of the invention. Multichannel coordinated infusion system is typically used for continuous renal replacement therapies (CRRT) such as continuous arteriovenous hemofiltration (CAVH) or continuous arteriovenous hemodialysis (CAVHD) and is essentially a bed site replacement for a normal hemodialysis procedure.

Patients with chronic renal failure usually make use of a normal hemodialysis procedure. Hemodialysis is often very difficult for the patient, as there is a large amount of fluid shifts and electrolyte changes as well as many physiologic changes that occur while the patient is having his or her blood cleansed by a hemodialysis machine. The procedure generally drains the patient's energy making him/her tired and weak. Such side effects are even more pronounced with patients who are critically ill. Patient's bodies often cannot tolerate the procedure as their blood pressure drops and they have other physiologic problems. To overcome the extreme nature of hemodialysis, the CRRT (CAVH and CAVHD) procedures were developed. CRRT is similar to hemodialysis, except that the patient is on the machine (the filter system) continuously rather than for several hours at varying increments of time. In CRRT, it is important to keep accurate records of dialysis liquids and intravenous liquids entering the patient and the amount of liquids leaving the patient. That is, a mass balance must exist when liquids are drawn and replaced from a patient. Severe clinical problems, and even death, may occur if these fluid balances are not carefully regulated. The advantage of these therapies is that they are less stressful on the body and provide continuous treatment as opposed to three to four hour hemodialysis sessions.

The goal of these procedures is the same as hemodialysis, to clean the blood, but the process is more gradual. CAVH typically uses the patient's arterial blood pressure to deliver blood to a low-resistance hemofilter. To maintain systemic blood pressure, the patient receives replacement fluids. CAVHD is a modification of the CAVH method that uses an infusion pump to move a dialysate solution countercurrent to blood flow, adding the ability to continuously remove solute while removing fluid.

Both CAVH and CAVHD provide continuous renal replacement therapy, thus allowing removal of solutes and modification of the volume and composition of the extracellular fluid to occur evenly over time. Unstable patients, who are often intolerant of the abrupt fluid volume and solute concentration changes that accompany standard hemodialysis treatments, can usually be treated safely with CAVH or CAVHD.

The hemofiltration system utilizes a small filter that is highly permeable to water and small solutes, but impermeable to plasma proteins and the formed elements of the blood. The filter is placed in an extra corporeal circuit. As the blood perfuses through the hemofitter an ultrafiltrate of plasma is removed. The ultrafiltrate is concurrently replaced using a fluid with an electrolyte composition that is either similar to that ot normal plasma or specifically designed to correct abnormalities in the individual patient. The hemofiltration circuit connects a large artery and vein. Blood is typically pumped through the circuit by the heart, allowing the patient's arterial-to-venous pressure gradient to provide the pressure to drive the system. This system however does not accurately control the fluid mass balance discussed above.

In the present invention, a dialysis filter 144 is connected between an arterial venous ("AV") shunt on one side and two weight cells and LVP combination modules 146 and 148 on the other. The LVP combination modules are both controlled by a single PCU 150. Blood 152 from the patient enters the filter and an ultrafiltrate 154 is removed and contained in receptacle 156, leaving only blood cellular components, and blood plasma proteins. A replacement ultrafiltrate 158 enters the filter 144 via a conduit 160 and is mixed with the separated blood plasma proteins before returning 162 the blood to the patient. The flow rate of the ultrafiltrate 154 removed from the patient is monitored by PCU 150 that controls the LVP module 146. Likewise, the flow rate of the ultrafiltrate 158 replaced into the patient is accurately controlled by the PCU 150 that controls the LVP module 104.

By doing so, the PCU 150 controls the LVP module 148 to accurately deliver an infusion into an IV line based on the flow rate desired. The LVP module 146 is programmed to accurately withdraw fluid from the line by measuring the amount of fluid withdrawn. The system can be programmed to maintain a preset difference between the two infusions to result in an accurate positive or negative balance. Similar to the embodiments described above, since the two infusion modules 146 and 148 are programmed by the same PCU 150, when one infusion module stops for any reason, the second module will also stop.

Improved accuracy, control and the ability to program either a positive or negative pressure differential in a CRRT system may be achieved using the multichannel coordinated infusion system 140 with weight cell capability and central PCU 150 control. The multichannel coordinated infusion system 140 is also able to overcome conditions such as high intake and output pressure differences that significantly affect volume of delivery or withdrawal.

FIG. 9 is a flow chart of a method 170 of infusing an ultrafiltrate into a patient. An infusion ultrafiltrate is first delivered to a hemofilter at step 172. A weight cell then measures at step 174 the amount of infusion ultrafiltrate in the infusion system. At the same time the patient's removed ultrafiltrate is received at step 176 from the hemofilter. The amount of removed ultrafiltrate in the infusion system is measured at step 178 after which the flow rates to and from the hemofilter are adjusted at step 180. The adjustment is based on the measured amounts of infusion and removed ultrafittrate in the infusion system and a predetermined desired pressure differential between the infusion and removed ultrafiltrates. The method 170 may be repeated 182 until the desired infusion ultrafiltrate is delivered or removed from the patient.

While the particular infusion systems and methods as herein shown and disclosed in detail are fully capable of performing as indicated and providing the advantages herein before stated, it is to be understood that they are merely illustrative of the presently preferred embodiments of the invention, and that no limitations are intended to the details of construction or design herein shown other than as described in the appended claims.

### Features of the parent application include:

1. An infusion system, comprising:
   a first delivery unit that delivers a first infusate to an infusion circuit;
   a second delivery unit that delivers a second infusate to the infusion circuit;
   a measurement device that determines an amount of undelivered first infusate remaining to be infused; and
   a control system that:
      controls delivery of the first infusate from the first delivery unit and delivery of the second infusate from the second delivery unit;
         enables input of a predetermined volume of the first infusate to be delivered and input of a predetermined time period for infusion of the predetermined first insufate volume;
      determines a time remaining in the predetermined infusion time period;
      communicates with the first delivery unit, the second delivery unit, and the measurement device to determine an optimum first infusate infusion flow rate based on the predetermined first infusate volume, the predetermined infusion time period, the determined amount of undelivered first infusate remaining in the system and the determined time remaining in the predetermined infusion time period; and
      controls the first delivery unit to deliver the first infusate at the determined optimum first infusate infusion flow rate.
2. An infusion system as recited in feature 1 wherein the first infusate is a medication and the second infusate is a neutral carrying solution.
3. An infusion system as recited in feature 1 wherein the control system stops and restarts both of the first and second delivery units simultaneously.
4. An infusion system as recited in feature 1 further comprising a programming unit for providing an interface between the infusion system and a user of the infusion system.
5. An infusion system as recited in feature 1 wherein the control system automatically recalculates the optimum flow rate after any interruptions of either the first or the second pump units.
6. An infusion system as recited in feature 1 wherein the first delivery unit comprises a syringe pump.
7. An infusion system as recited in feature 1 wherein the first delivery unit comprises an infusion pump.
8. An infusion system as recited in feature 1 wherein the first delivery unit comprises a receptacle containing the first infusate, and the measurement device comprises a weight cell for ascertaining the weight of the first infusate containing receptacle.
9. An infusion system as recited in feature 5 wherein the control system is located within the programming unit.
10. An infusion system as recited in feature 6 wherein the syringe pump includes a syringe, and the infusion system further comprises a syringe size detection system for detecting the total volume of the syringe.
11. An infusion system as recited in feature 6 wherein the syringe pump includes a syringe that includes a plunger and the measurement device comprises a position sensor for sensing a position of the plunger of the syringe in the syringe pump.
12. An infusion system as recited in feature 8 wherein the amount of first infusate remaining in the system is a weight of first infusate remaining in the infusion system and is calculated from the weight of the first infusate containing receptacle less a tare weight of the receptacle.
13. An infusion system as recited in feature 11 wherein the amount of first infusate remaining in the system is a volume of first infusate remaining in the infusion system and is calculated from the position of the plunger and a total volume of the syringe.
14. An infusion system as recited in feature 12 wherein the optimum infusion flow rate is calculated from the weight of the first infusate remaining in the infusion system, and the first infusate's specific gravity, and the time remaining in the predetermined infusion time period.
15. An infusion system as recited in feature 14 wherein the infusion system further comprises a receptacle detection system for detecting the type and tare weight of the receptacle.
16. An infusion system comprising:
   a first delivery unit that delivers an infusion ultrafiltrate to a hemofilter;
   a first measurement device that determines an amount of infusion ultrafiltrate in the infusion system;
   a receiver unit that receives a patient's removed ultrafiltrate from the hemofilter;
   a second measurement device that determines an amount of removed ultrafiltrate in the infusion system;
   a control system communicating with the delivery and receiver units and the first and second measurement units to determine optimum flow rates to and from the hemofilter, based upon the determined amounts of infusion ultrafiltrate and removed ultrafiltrate in the infusion system, and a predetermined desired pressure differential between the infusion and removed ultrafiltrates, and for controlling the delivery and receiver units accordingly.
17. An infusion system as recited in feature 16 wherein the delivery and receiver units comprise infusion pumps.
18. An infusion system as recited in feature 16 wherein the control system stops and restarts both of the delivery and receiver units simultaneously.
19. An infusion system as recited in feature 16 further comprising a programming unit for providing an interface between the infusion system and a user of the infusion system.
20. An infusion system as recited in feature 16 wherein the control system automatically recalculates the optimum flow rates after any interruptions of either the delivery or the receiver units.
21. An infusion system as recited in feature 17 wherein the delivery unit includes an infusion receptacle for containing the infusion ultrafiltrate, the receiver unit includes a removal receptacle for containing the removed ultrafiltrate, and the first and second measurement devices comprise weight cells for ascertaining the weight of the infusion ultrafiltrate contained in the infusion receptacle and the removed ultrafiltrate contained in the removal receptacle.
22. An infusion system as recited in feature 19 wherein the control system is located within the programming unit.
23. An infusion system as recited in feature 21 wherein the weight of the infusion and removed ultrafiltrates in the infusion system is calculated from the weight of each of the infusion and removal receptacles less a tare weight of each of the infusion and removal receptacles.
24. An infusion system as recited in feature 21 wherein the infusion system further comprises a receptacle detection system for detecting the type and tare weight of each of the infusion and removal receptacles.
25. A method of infusing a second infusate into a patient, comprising the steps of:
   determining a total volume of first infusate is to be infused;
   ascertaining a period over which the total volume of first infusate is to be infused;
   detecting a remaining amount of first infusate to be infused;
   calculating time remaining in the period;
   delivering a second infusate into an infusion circuit;
   infusing the first infusate into the infusion circuit; and
   automatically adjusting the infusion based on the remaining amount of first infusate to be infused and the time remaining in the period.
26. A method as recited in feature 25 wherein the method further comprises the step of repeating the detecting, calculating, delivering, infusing and automatically adjusting steps until the total volume of first infusate is infused.
27. A method as recited in feature 25 wherein the determining step comprises the step of receiving an input from a user of the total volume of first infusate to be infused.
28. A method as recited in feature 25 wherein the determining step comprises the steps of recognizing characteristics of a receptacle in which the first infusate is contained, and calculating the total volume of first infusate to be infused from the receptacle characteristics.
29. A method as recited in feature 25 wherein the ascertaining step comprises the step of receiving an input from a user of the period over which the total volume of first infusate is to be infused.
30. A method as recited in feature 25 wherein the detecting step comprises the steps of establishing the position of a plunger of a syringe placed in a syringe pump, and calculating the remaining amount of first infusate to be infused from the plunger position and a total volume of the syringe.
31. A method as recited in feature 25 wherein the detecting step comprises the steps of ascertaining the weight of a first infusate containing receptacle, and calculating the remaining amount of first infusate to be infused from the weight of the first infusate containing receptacle.
32. A method as recited in feature 25 wherein the delivering step comprises pumping the second infusate via an infusion pump.
33. A method as recited in feature 25 wherein the infusing step comprises pumping the second infusate via an infusion pump.
34. A method as recited in feature 25 wherein the infusing step comprises pumping the first infusate via a syringe pump.
35. A method as recited in feature 25 wherein the automatically adjusting step further comprises stopping and restarting both the delivering and infusing steps simultaneously after any interruptions.
36. A method as recited in feature 25 wherein the automatically adjusting step further comprises automatically recalculating the infusion after any interruptions.
37. A method of infusing an ultrafiltrate into a patient, comprising the steps of:
   delivering an infusion ultrafiltrate to a hemofilter;
   measuring the amount of infusion ultrafiltrate in the infusion system;
   receiving a patient's removed ultrafiltrate from the hemofilter;
   measuring the amount of removed ultrafiltrate in the infusion system; and
   adjusting flow rates of the delivering and the receiving steps based upon the measured amounts of infusion and removed ultrafiltrate in the infusion system and a predetermined desired pressure differential between the infusion and removed ultrafiltrates.
38. A method as recited in feature 37 wherein the measuring step comprises weighing receptacles containing the ultrafiltrate.
39. A method as recited in feature 37 wherein the method further comprises the step of repeating the method until a desired ultrafiltrate is delivered or removed from a patient.

## Claims

1. An infusion system comprising:
a first delivery unit that delivers an infusion ultrafiltrate to a hemofilter;
a first measurement device that determines an amount of infusion ultrafiltrate in the infusion system;
a receiver unit that receives a patient's removed ultrafiltrate from the hemofilter; a second measurement device that determines an amount of removed ultrafiltrate in the infusion system; and
a control system communicating with the delivery and receiver units and the first and second measurement units to determine optimum flow rates to and from the hemofilter, based upon the determined amounts of infusion ultrafiltrate and removed ultrafiltrate in the infusion system, and a predetermined desired pressure differential between the infusion and removed ultrafiltrates, and for controlling the delivery and receiver units accordingly.

2. An infusion system as recited in claim 1, wherein the delivery and receiver units comprise infusion pumps.

3. An infusion system as recited in claim 2, wherein the delivery unit includes an infusion receptacle for containing the infusion ultrafiltrate, the receiver unit includes a removal receptacle for containing the removed ultrafiltrate, and the first and second measurement devices comprise weight cells for ascertaining the weight of the infusion ultrafiltrate contained in the infusion receptacle and the removed ultrafiltrate contained in the removal receptacle.

4. An infusion system as recited in claim 3, wherein the weight of the infusion and removed ultrafiltrates in the infusion system is calculated from the weight of each of the infusion and removal receptacles less a tare weight of each of the infusion and removal receptacles.

5. An infusion system as recited in claim 3 or 4, wherein the infusion system further comprises a receptacle detection system for detecting the type and tare weight of each of the infusion and removal receptacles.

6. An infusion system as recited in any of the preceding claims, wherein:
the control system stops and restarts both of the delivery and receiver units simultaneously; and/or
the control system automatically recalculates the flow rates after any interruptions of either the delivery or the receiver units.

7. An infusion system as recited in any of the preceding claims, further comprising a programming unit for providing an interface between the infusion system and a user of the infusion system.

8. An infusion system as recited in claim 7, wherein the control system is located within the programming unit.

9. A method of infusing an ultrafiltrate into a patient, comprising the steps of:
delivering an infusion ultrafiltrate to a hemofilter;
measuring the amount of infusion ultrafiltrate in the infusion system;
receiving a patient's removed ultrafiltrate from the hemofilter;
measuring the amount of removed ultrafiltrate in the infusion system; and
adjusting flow rates of the delivering and the receiving steps based upon the measured amounts of infusion and removed ultrafiltrate in the infusion system and a predetermined desired pressure differential between the infusion and removed ultrafiltrates.

10. A method as recited in claim 9 wherein:
the measuring step comprises weighing receptacles containing the ultrafiltrate; and/or
the method further comprises the step of repeating the method until a desired ultrafiltrate is delivered or removed from a patient.

11. An infusion system, comprising:
a first delivery unit that delivers a first infusate to an infusion circuit;
a second delivery unit that delivers a second infusate to the infusion circuit;
a measurement device that determines an amount of undelivered first infusate remaining to be infused; and
a control system that:
controls delivery of the first infusate from the first delivery unit and delivery of the second infusate from the second delivery unit;
enables input of a predetermined volume of the first infusate to be delivered and input of a predetermined time period for infusion of the predetermined first insufate volume;
determines a time remaining in the predetermined infusion time period;
communicates with the first delivery unit, the second delivery unit, and the measurement device to determine an optimum first infusate infusion flow rate based on the predetermined first infusate volume, the predetermined infusion time period, the determined amount of undelivered first infusate remaining in the system and the determined time remaining in the predetermined infusion time period; and
controls the first delivery unit to deliver the first infusate at the determined optimum first infusate infusion flow rate.

12. An infusion system as recited in claim 11, wherein:
the first infusate is a medication and the second infusate is a neutral carrying solution; and/or
the control system stops and restarts both of the first and second delivery units simultaneously; and/or
the infusion system further comprises a programming unit for providing an interface between the infusion system and a user of the infusion system; and/or
the control system automatically recalculates the optimum flow rate after any interruptions of either the first or the second pump units; and/or
the first delivery unit comprises one of: a syringe pump and an infusion pump;
and/or
the first delivery unit comprises a receptacle containing the first infusate, and the measurement device comprises a weight cell for ascertaining the weight of the first infusate containing receptacle.

13. An infusion system as recited in claim 11, wherein:
the infusion system further comprises a programming unit for providing an interface between the infusion system and a user of the infusion system, wherein the control system is located within the programming unit; and/or
the first delivery unit comprises a syringe pump and the infusion system further comprises one of a syringe size detection system for detecting the total volume of the syringe and a position sensor for sensing a position of the plunger of the syringe in the syringe pump; and/or
the first delivery unit comprises a receptacle containing the first infusate, and the measurement device comprises a weight cell for ascertaining the weight of the first infusate containing receptacle and wherein the amount of first infusate remaining in the system is a weight of first infusate remaining in the infusion system and is calculated from the weight of the first infusate containing receptacle less a tare weight of the receptacle; and/or
the optimum infusion flow rate is calculated from the weight of the first infusate remaining in the infusion system, and the first infusate's specific gravity, and the time remaining in the predetermined infusion time period; and/or
the infusion system further comprises a receptacle detection system for detecting the type and tare weight of the receptacle.

14. A method of infusing a second infusate into a patient, comprising the steps of:
determining a total volume of first infusate is to be infused;
ascertaining a period over which the total volume of first infusate is to be infused;
detecting a remaining amount of first infusate to be infused;
calculating time remaining in the period;
delivering a second infusate into an infusion circuit;
infusing the first infusate into the infusion circuit; and
automatically adjusting the infusion based on the remaining amount of first infusate to be infused and the time remaining in the period.

15. A method as recited in claim 17, wherein the method further comprises the step of repeating the detecting, calculating, delivering, infusing and automatically adjusting steps until the total volume of first infusate is infused and/or one of the following:
the determining step comprises receiving an input from a user of the total volume of first infusate to be infused;
the determining step comprises recognizing characteristics of a receptacle in which the first infusate is contained, and calculating the total volume of first infusate to be infused from the receptacle characteristics;
the ascertaining step comprises the step of receiving an input from a user of the period over which the total volume of first infusate is to be infused;
the detecting step comprises the steps of establishing the position of a plunger of a syringe placed in a syringe pump, and calculating the remaining amount of first infusate to be infused from the plunger position and a total volume of the syringe;
the detecting step comprises the steps of ascertaining the weight of a first infusate containing receptacle and calculating the remaining amount of first infusate to be infused from the weight of the first infusate containing receptacle;
the delivering step comprises pumping the second infusate via an infusion pump;
the infusing step comprises pumping the second infusate via a syringe pump;
the automatically adjusting step further comprises stopping and restarting both the delivering and infusing steps simultaneously after any interruptions; and
the automatically adjusting step further comprises automatically recalculating the infusion after any interruptions.
